# EUROPEAN PATENT APPLICATION

(11) **EP 2 145 599 A1**
(43) Date of publication of application: **20.01.2010**
(21) Application number: 09008874.1
(22) Date of filing: 07.07.2009
(51) Int. Cl.: A61C 5/06, B05C 17/005

(54) **Mixing tip**

(30) Priority: 15.07.2008 JP 2008183942
(71) Applicant: GC Corporation, Itabashi-ku Tokyo 174-8585 (JP)
(72) Inventor: Miyano, Tatsunosuke, Tokyo (JP); Takahashi, Masayuki, Tokyo (JP); Noguchi, Kazuma, Soka-City, Saitama (JP)
(74) Representative: Müller-Boré & Partner Patentanwälte

(57) **Abstract**

A mixing tip for mixing two kinds of pastes, to be locked and fixed on a syringe holder, the mixing tip includes a housing (2) internally having a mixing element (1), a paste guiding member (3) being fixed to an end part positioned at the opposite side to an ejection port of the housing (2), and a locking member having a pawl part to be locked with a locking hole formed on the syringe holder and a projection part to be inserted to a hole part bored on the syringe holder, or has a pair of pawl parts to be inserted to and locked with two locking holes formed on the syringe holder, and the housing (2) and the paste guiding member (3) are made of a colored plastic material not transmitting a light having a wavelength photo-activating the photopolymerization catalyst.

## Description

The present invention relates to a mixing tip capable of being easily, certainly locked and fixed on a cylinder holder in which two syringes are fixed in parallel. The two syringes store two kinds of pastes of an adhesive for a dental bracket including a resin-reinforced glass ionomer cement, in which at least one of the pastes includes a photopolymerization catalyst. Further, the mixing tip is made of a material capable of preventing that the photopolymerization catalyst is photo-activated so as to photo-cure the pastes in the mixing tip or shorten a curing time, in a state that the both pastes are flowed in and kneaded in the mixing tip. Furthermore, the mixing tip is made of a material in which a dentist can visually confirm the pastes flowed in and kneaded in the mixing tip.

In a dental treatment field, a resin-reinforced photopolymerization type glass ionomer cement broadly is used as an adhesive for an orthodontic bracket because of having biocompatibility, aesthetic property, and deterioration prevention due to water content at a time of initial curing. In recent years, as for the resin-reinforced photopolymerization type glass ionomer cement, for example, Japanese Patent Application Laid-Open No. 11-228327 discloses a pasty glass ionomer cement, which includes a first paste obtained by mixing a fluoroaluminosilicate glass powder and a polymerizable monomer not having an acid group and a second paste including an α-β unsaturated carboxylic acid polymer aqueo.us solution and a filler not reacting with the α-β unsaturated carboxylic acid polymer. In this pasty glass ionomer cement, at least one of the both pasts includes a photopolymerization catalyst and the both pastes are mixed and kneaded just before using.

When the two kinds of the pastes of the pasty resin-reinforced photopolymerization type glass ionomer cement are mixed, a static mixing tip is usually used for mixing the pastes respectively extruded from syringes in which the pastes are stored. However, when the mixing tip is exposed to light for a comparatively long time, the photopolymerization catalyst included in the paste is photo-activated, and thus the paste in the mixing tip may be photo-cured, or a curing time may be shortened. Thus, it is necessary that the mixing tip is made of a material which does not transmit a light having a wavelength which photo-activates the photopolymerization catalyst.

Further, when a dentist uses this pasty resin-reinfored photopolymerization type glass ionomer cement as an adhesive for an orthodontic blanket, the dentist needs to visually confirm whether respective pastes extruded from the syringes are certainly poured in the mixing tip and to guess how long it will take until the respective pastes poured in the mixing tip are kneaded and pushed out from a top end of the mixing tip. Thus, the mixing tip needs to be made of such a material that the inside can be visually confirmed by the dentist.

Further, since the mixing tip.should be locked and fixed on a top end of a syringe holder in which two syringes are fixed in parallel, it is necessary that the mixing tip is easily locked and fixed on the syringe holder. For example, Japanese Patent Application Laid-Open No. 8-276125 discloses such a mixing tip in Fig. 18 . In this invention, a mixing tip (a mixer) is formed to have an insertion projection on an outer face of a lower end thereof, and a syringe holder (a cartridge) is formed to have an insertion piece for receiving the insertion projection of the mixing tip. The insertion projection of the mixing tip is made in contact with the insertion piece of the syringe holder so as to cross with the insertion piece, and then the mixing tip is rotated by approximately 90° so that the insertion projection is locked and fixed with the insertion piece. However, the mixing tip is not certainly locked because of only being locked by rotating, so that any prevention mechanism is necessary to prevent the mixing tip from being removed.

Further, Japanese Patent Application Laid-Open No. 4-239477 discloses a cartridge to more certainly lock and fix the mixing tip. In this invention, top end portions of two syringes respectively have male screws which constitute one male screw when both the syringes are coupled together. While the mixing tip is made in contact with the top end portions of both the syringes respectively having the male screw, a coupling nut is screwed in so as to cover the mixing tip and the syringes, and thereby the mixing tip and the syringes are connected. However, since the coupling nut should be screwed to the male screw portion in case of this cartridge, it takes time and effort to mount the mixing tip. Further, a specific processing is necessary to form the male screws which constitute one male screw when laying a plurality of the syringes are coupled together.

The present invention is directed to solve the aforementioned problems of the conventional technologies and provide a mixing tip which is made of a material capable of preventing the problem that a photopolymerization catalyst included in at least one of two kinds of pastes to make an adhesive for a dental bracket consisting of a resin-reinforced glass ionomer cement is photo-activated so as to photo-cure the pastes in the mixing tip or shorten a curing time, wherein the material enables a dentist to visually confirm the pastes in the mixing tip, and which can be easily, certainly locked and fixed on a syringe holder.

Present inventors carried out earnest works to solve the aforementioned problems and, as a result, they found out the followings to complete the present invention. A mixing tip is locked at a top end of a syringe holder having two parallel syringes storing two kinds of pastes as a first paste and a second paste, which are used for an adhesive for an orthodontic bracket. The first paste includes at least an α-β unsaturated carboxylic acid polymer, water, and a filler not reacting with the α-β unsaturated carboxylic acid polymer. The second paste includes at least a fluoroaluminosilicate glass powder and a polymerizable monomer not having an acid group. One or both of the first and second pastes include a photopolymerization catalyst. The mixing tip includes a housing internally having a mixing element; a paste guiding member being fixed with an end part positioned at the opposite side to an ejection port of the housing and including a pair of paste inlets attached respectively to nozzles of the two syringes and flow path parts for guiding the pastes extruded from the respective syringes into the housing from the paste inlets; and a locking member being fixed on or integrally formed on the paste guiding member or the housing, where the locking member has a pawl part to be locked with a locking hole formed on the syringe holder and a projection part to be inserted to a hole part bored on the syringe holder or has a pair of pawl parts to be inserted to and locked with two locking holes formed on the syringe holder. In the mixing tip, the housing and the paste guiding member are made of a colored plastic material, which does not transmit a light having a wavelength photo-activating the photopolymerization catalyst but transmits light except the light having the photo-activating wavelength. Since the mixing tip is made of the colored plastic material not transmitting the light having a wavelength photo-activating the photopolymerization catalyst but transmitting a light except the light having the photo-activating wavelength, it can be prevented that the photopolymerization catalyst included in at least one of the two kinds of the pastes used for the adhesive for a dental bracket is photo-activated in the mixing tip so as to photo-cure the adhesive for a dental bracket or shorten a curing time. Further, a dentist can visually confirm the pastes in the mixing tip. Furthermore, since the mixing tip is locked with the two locking members only by being inserted to the syringe holder, the mixing tip can be remarkably easily fixed and locked. In addition, the two locking members have the pawl part to be locked with the locking hole formed on the syringe holder and the projection part to be inserted to the hole part bored on the syringe holder, or have a pair of the pawl parts to be locked with the locking holes formed on the syringe holder. Thus, the mixing tip can be certainly locked and fixed on the syringe holder by the pawl parts.

According to an aspect of the present invention, a mixing tip is locked at a top end of a syringe holder having two parallel syringes storing two kinds of pastes as a first paste and a second paste, which are used for an adhesive for an orthodontic bracket. The first paste includes at least an α-β unsaturated carboxylic acid polymer, water, and a filler not reacting with the α-β unsaturated carboxylic acid polymer. The second paste includes at least a fluoroaluminosilicate glass powder and a polymerizable monomer not having an acid group. One or both of the first and second pastes include a photopolymerization catalyst. The mixing tip includes a housing internally having a mixing element; a paste guiding member being fixed to an end part positioned at the opposite side to an ejection port of the housing and including a pair of paste inlets attached respectively to nozzles of the two syringes and flow path parts for guiding the pastes extruded from the respective syringes into the housing from the paste inlets; and a locking member being fixed on or integrally formed on the paste guiding member or the housing, and having a pawl part to be locked with a locking hole formed on the syringe holder and a projection part to be inserted to a hole part bored on the syringe holder, or having a pair of pawl parts to be inserted to and locked with two locking holes formed on the syringe holder. The housing and the paste guiding member are made of a colored plastic material, which does not transmit a light having a wavelength photo-activating the photopolymerization catalyst but transmits light except the light having the photo-activating wavelength.

Further, when each pawl part includes a releasing mechanism for releasing the locking state of the syringe holder and the pawl part, the mixing tip can be easily exchanged, so it is preferable.

A mixing tip of the present invention is locked at a top end of a syringe holder having two parallel syringes storing two kinds of pastes as a first paste and a second paste, which are used for an adhesive for an orthodontic bracket. The first paste includes at least an α-β unsaturated carboxylic acid polymer, water, and a filler not reacting with the α-β unsaturated carboxylic acid polymer. The second paste includes at least a fluoroaluminosilicate glass powder and a polymerizable monomer not having an acid group. One or both of the first and second pastes include a photopolymerization catalyst. The mixing tip includes a housing internally having a mixing element; a paste guiding member being fixed to an end part positioned at the opposite side to an ejection port of the housing and including a pair of paste inlets attached respectively to nozzles of the two syringes and flow path parts for guiding the pastes extruded from the respective syringes into the housing from the paste inlets; and a locking member being fixed on or integrally formed on the paste guiding member or the housing, and having a pawl part to be locked with a locking hole formed on the syringe holder and a projection part to be inserted to a hole part bored on the syringe holder, or having a pair of pawl parts to be inserted to and locked with two locking holes formed on the syringe holder. Therefore, the mixing tip can be remarkably easily and certainly locked and fixed only by being inserted to the syringe holder. Further, the mixing tip is made of the colored plastic material not transmitting a light having a wavelength photo-activating the photopolymerization catalyst but transmitting light except the light having the photo-activating wavelength. Thus, even when the mixing tip is exposed to light for long time, it can be prevented that the photopolymerization catalyst is photo-activated in the mixing tip so as to photo-cure the adhesive for a dental bracket or shorten a curing time. Furthermore, a dentist can visually confirm the pastes in the mixing tip.

Further, when each pawl part includes a releasing mechanism for releasing the locking state of the syringe holder and the pawl part, the mixing tip can be easily exchanged.
Fig. 1 is a perspective explanatory view for illustrating a state that one example of a mixing tip according to the present invention is disassembled.
Fig. 2 is a side view for illustrating a state before the mixing tip according to the present invention of Fig. 1 is locked and fixed on a syringe holder.
Fig. 3 is a side view for illustrating a state that the mixing tip according to the present invention of Fig. 1 is locked and fixed on a syringe holder.
Fig. 4 is an enlarged perspective view for illustrating a pawl part of a mixing tip according to the present invention.
Fig. 5 is a perspective view for illustrating a locking hole of a syringe holder with which a pawl part of the mixing tip according to the present invention is locked.
Fig. 6 is a side view for illustrating another example of a mixing tip according to the present invention having two pawl parts as a locking member.
Fig. 7 is a side view for illustrating yet another example of a mixing tip according to the present invention.
Fig. 8 is an enlarged perspective view for illustrating a releasing mechanism formed at a pawl part of a mixing tip according to the present invention.
Fig. 9 is an explanatory view for illustrating a state that a mixing tip according to the present invention is removed.
Fig. 10 is a sectional explanatory view for illustrating an internal state of a paste guiding member.

A mixing tip according to the present invention will be described in detail below with reference to drawings.

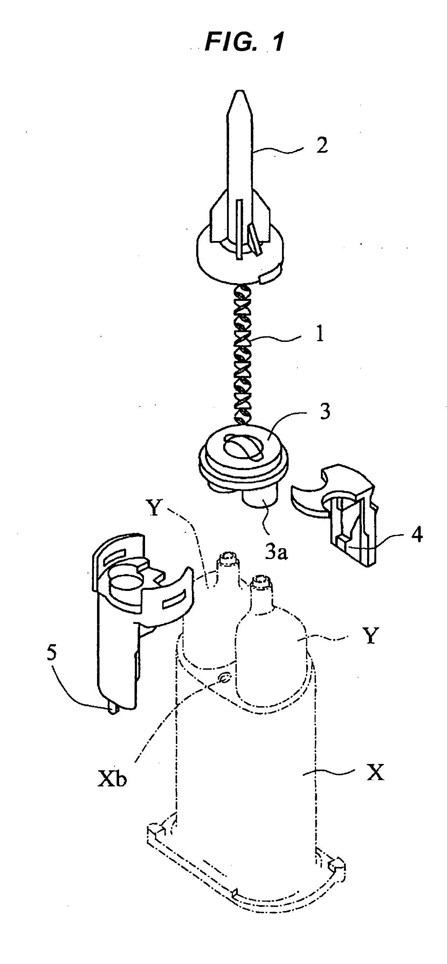

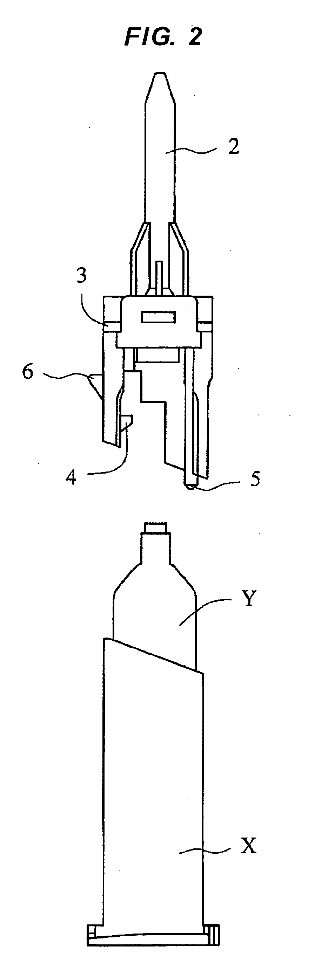

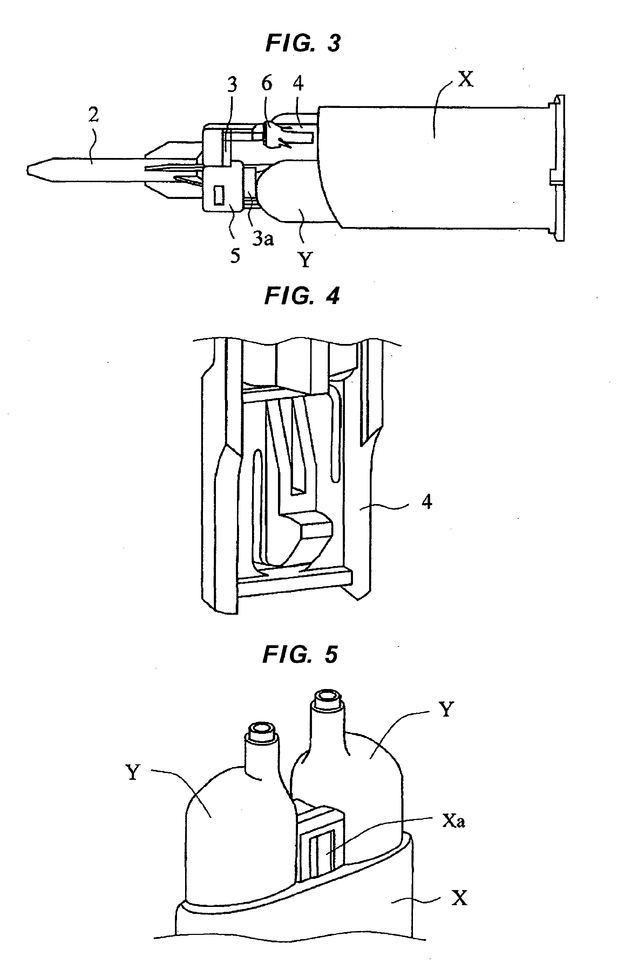

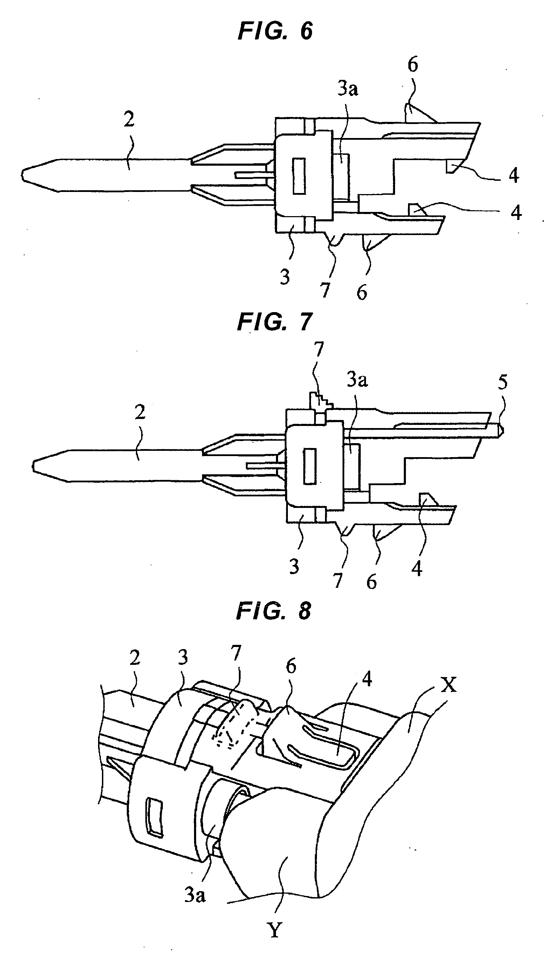

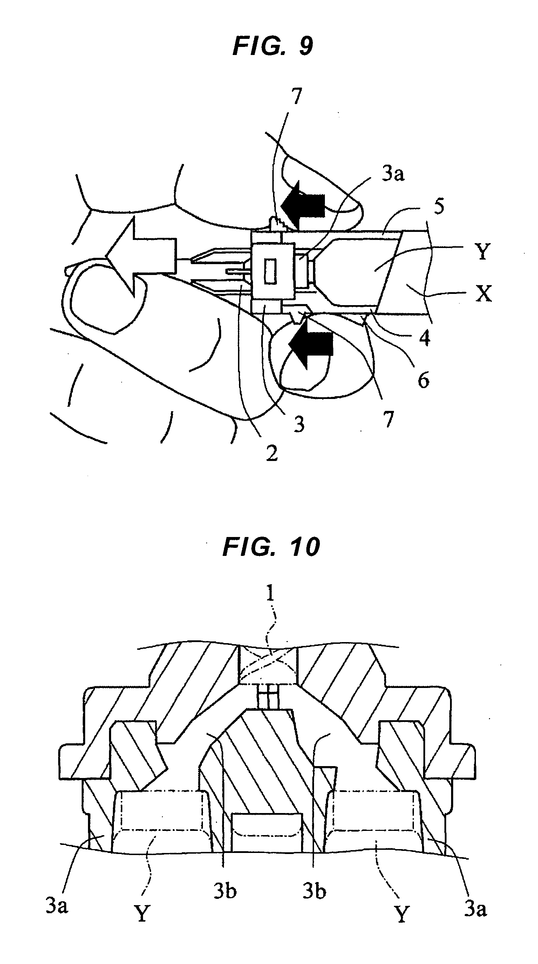

Fig. 1 is a perspective explanatory view for illustrating a state that one example of a mixing tip according to the present invention is disassembled. Fig. 2 is a side view for illustrating a state before the mixing tip according to the present invention of Fig. 1 is locked and fixed on a syringe holder. Fig. 3 is a side view for illustrating a state that the mixing tip according to the present invention of Fig. 1 is locked and fixed on a syringe holder. Fig. 4 is an enlarged perspective view for illustrating a pawl part of a mixing tip according to the present invention. Fig. 5 is a perspective view for illustrating a locking hole of a syringe holder with which a pawl part of the mixing tip according to the present invention is locked. Fig. 6 is a side view for illustrating another example of a mixing tip according to the present invention having two pawl parts as a locking member. Fig. 7 is a side view for illustrating yet another example of a mixing tip according to the present invention. Fig. 8 is an enlarged perspective view for illustrating a releasing mechanism formed at a pawl part of a mixing tip according to the present invention. Fig. 9 is an explanatory view for illustrating a state that a mixing tip according to the present invention is removed. Fig. 10 is a sectional explanatory view for illustrating an internal state of a paste guiding member.

In the drawings, a mixing element 1 is internally provided in a housing 2. The mixing element 1 is not limited especially if it can fully mix a first paste and a second paste, which compose for an adhesive for an orthodontic bracket. The first paste includes at least an α-β unsaturated carboxylic acid polymer, water, and a filler not reacting with the α-β unsaturated carboxylic acid polymer. The second paste includes at least a fluoroaluminosilicate glass powder and a polymerizable monomer not having an acid group. One or both of the first and second pastes include a photopolymerization catalyst.

The housing 2 is made of a colored plastic material, which does not transmit a light having a wavelength photo-activating the photopolymerization catalyst but transmits light except the light having the photo-activating wavelength. The wavelength is within a range of 450 nm or more and 490 nm or less, for example, when a combination of camphorquinone and tertiary amine is used as the photopolymerization catalyst. The wavelength is within a range of 370 nm or more and 410 nm or less, when acyl phosphine oxide is used as the photopolymerization catalyst. Therefore, the colored plastic material not transmitting the light having a wavelength, which photo-activates the photopolymerization catalyst, should be selected corresponding to the photopolymerization catalyst to be used. Thus, even when the mixing tip is exposed to light for a comparatively long time, it can be prevented that the photopolymerization catalyst included in the pastes in the housing 2 is photo-activated so as to photo-cure the pastes or shorten a curing time. For example, when a combination of camphorquinone and tertiary amine is used as the photopolymerization catalyst, a plastic material having a yellowish color is selected. Further, since such a colored plastic material transmits light except the light having a wavelength photo-activating the photopolymerization catalyst, a dentist can visually confirm whether respective pastes extruded from two syringes Y and Y are certainly poured into the mixing tip, or can guess how long it will take until the respective pasts poured into the mixing tip are kneaded and pushed out from a top end of the mixing tip.

A paste guiding member 3 is made of a colored plastic material, which does not transmit a light having a wavelength photo-activating the photopolymerization catalyst but transmits light except the light having the photo-activating wavelength. The paste guiding member 3 includes paste inlets 3a attached to nozzles of the two syringes Y and Y, and flow path parts 3b for guiding the pastes extruded from the respective syringes Y and Y into the housing 2 from the paste inlets 3a. The paste guiding member 3 is fixed to an end part positioned at the opposite side to an ejection port of the housing 2. The flow path parts 3b in the paste guiding member 3 are flow paths for connecting the syringes Y and Y and the housing 2. By appropriately designing a cross-sectional area ratio or a volume ratio of flow path parts 3b, a kneaded material having a proper ratio can be obtained from the beginning of kneading.

A pawl part 4 and a projection part 5 are locking members for locking and fixing to the syringe holder X, and fixed or integrally formed at the paste guiding member 3 or the housing 2. The pawl part 4 is locked with a locking hole Xa formed on the syringe holder X, and the projection part 5 is inserted into a hole part Xb bored at the syringe holder X. A mixing tip according to the present invention includes such the two locking members. In one case, the two locking members are the pawl part 4 to be locked with the locking hole Xa formed on the syringe holder X and the projection part 5 to be inserted into the hole part Xb bored at the syringe holder X. In another case, the two locking members are a pair of the pawl parts 4 and 4 to be locked with the locking holes Xa and Xa formed on the syringe holder X. In these cases, at least one of the two locking members is the pawl part 4, so that the mixing tip can be certainly locked and fixed on the syringe holder X.

In the embodiment in Fig. 2, the locking members are the pawl part 4 and the projection part 5 respectively. The pawl part 4 is convex inwardly so as to be locked with the locking hole Xa formed on the syringe holder X. On the other hand, the projection part 5 is only inserted into the hole part Xb bored at the syringe holder X. Since the locking members are thus locked at two points, the mixing tip according to the present invention can be certainly locked and fixed without jouncing. Further, when the two locking members are the pawl parts 4 and 4 as illustrated in Fig. 6, the mixing tip can be locked and fixed more certainly.

A releasing mechanism 6 is formed on the pawl part 4 for releasing the locking state of the syringe holder X and the pawl part 4. Only the pawl part 4 is locked so as not to be removed from the syringe holder X among these two locking members. Thus, when the releasing mechanism 6 releases the locking state of the pawl part 4, the mixing tip can be removed easily.

When the mixing tip according to the present invention is produced, it is hard to integrally form the mixing element 1 and the paste guiding member 3, since the mixing element 1 has internally the housing 2. Thus, the mixing element 1 and the paste guiding member 3 are formed separately.

On the other hand, all of the paste guiding member 3 and the two locking members can be integrally formed, at least two of those can be integrally formed, or all of those can be formed separately so as to be fixed together as illustrated in Fig. 1.

When the mixing tip according to the present invention is locked and fixed on the top end of the syringe holder X, the mixing tip according to the present invention is only pushed-in toward the top end of the syringe holder X, in which the two syringes Y and Y are fixed in parallel, so as to be easily locked and fixed, as illustrated in Fig. 2.

In the embodiment in Fig. 2, the mixing tip according to the present invention has a shape in which the projection part 5 is projected more than the pawl part 4, and the syringe holder X also has a shape in which the top end side is obliquely cut corresponding to the shape of the projection part 5. Thus, when the mixing tip is going to be locked and fixed from the incorrect direction, a user can recognize the error instantly, so the mixing tip is not damaged.

As for the mixing tip according to the present invention, as illustrated in Fig. 3, the paste inlets 3a of the paste guiding member 3 are directly attached to the nozzles of the syringes Y and Y. However, as for a conventional mixing tip described in Japanese Patent Application Laid-Open No. 8-276125, since the mixing tip should be rotated to be mounted, the paste inlets cannot be directly attached to the nozzles of the syringes. Therefore, in the conventional technique, pastes straightly extruded from the nozzles of the syringes flow simply into the inside of the paste guiding member. Thus, when the inside of the mixing tip as a flow path is formed to have an angular shape, only the flow of a paste having high viscosity is interrupted. Further, when the flow path is straight, only a paste having low viscosity reaches the housing ahead of the other. Therefore, there is a problem that a kneaded material having a proper ratio can be hardly obtained.

On the other hand, in the mixing tip according to the present invention, the first paste and the second pastes extruded from the nozzles of the syringes Y and Y are directly guided to the flow path parts 3b as illustrated in Fig. 10, and thus the flowing direction of each paste can be easily changed immediately after extruding of the pastes. Therefore, there is no conventional problem that the flow of a paste straightly extruded is interrupted in the angular-shaped flow path. Further, by appropriately designing a cross-sectional area ratio or a volume ratio of each flow path part 3b, there is no problem that only a paste having low viscosity reaches the housing 2 ahead of the other. Thus, a kneaded material having proper ratio can be obtained from beginning of the kneading.

As for the mixing tip according to the present invention, two kinds of pastes including the first paste and the second paste which are used for an adhesive for an orthodontic bracket are mixed and kneaded for use. The first paste includes at least an α-β unsaturated carboxylic acid polymer, water, and a filler not reacting with the α-β unsaturated carboxylic acid polymer. The second paste includes at least a fluoroaluminosilicate glass powder and a polymerizable monomer not having an acid group. One or both of the first and second pastes include a photopolymerization catalyst. When the first and second pastes are mixed and kneaded, curing reaction proceeds slowly by an ionic reaction between the α-β unsaturated carboxylic acid polymer in the first paste and the fluoroaluminosilicate glass powder in the second paste. Then, the photopolymerization catalyst included in the paste extruded from the mixing tip is photo-activated by irradiating of the light from a light irradiator and the adhesive for an orthodontic bracket is cured in a short time in a state that the adhesive is applied to the surface of a tooth or the rear face of an orthodontic bracket. Then, the tooth and the orthodontic bracket can be adhered. Further, the housing 2 and the paste guiding member 3 are made of the colored plastic material, which does not transmit a light having a wavelength photo-activating the photopolymerization catalyst but transmits light except the light having the photo-activating wavelength. Thus, the mixed and kneaded adhesive for an orthodontic bracket is not photo-cured or not cured in a short time by photo-activating of the photopolymerization catalyst included in these pastes until the adhesive is extruded from the mixing tip.

Further, since an amount of the adhesive for an orthodontic bracket used for adhering the orthodontic bracket to the surface of a tooth is comparatively small, unused pastes remain in the syringes Y and Y. Thus, the unused pastes can be used later by exchanging the mixing tip. Therefore, it is very important for the present invention whether the used mixing tip can be exchanged easily or not.

Therefore, the mixing tip according to the present invention further includes the releasing mechanism 6 on at least one pawl part 4. Thus, the locking state of the syringe holder X and the pawl part 4 can be released easily, so it is preferable. For example, the releasing mechanism 6 can be formed by making the pawl part 4 to have a portion projected toward the outer surface side of the mixing tip and a groove part around the projected portion, pushing the projected portion, and thereby lifting a portion of the pawl part 4 locked with the locking hole Xa of the syringe holder X using the principle of the lever, as illustrated in Fig. 8. Then, as illustrated in Fig. 9, the mixing tip can be exchanged easily by pulling the mixing tip while pushing the projected portion. Furthermore, if a convex part 7 for hooking a finger is formed around the projected portion as illustrated in Figs. 7, 8, and 9, the mixing tip can be pulled more easily, so it is preferable.

## Claims

1. A mixing tip locked at a top end of a syringe holder having two parallel syringes,
wherein the two syringes store two kinds of a paste as a first paste and a second paste which are used for an adhesive for an orthodontic bracket,
wherein the first paste includes at least an α-β unsaturated carboxylic acid polymer, water, and a filler not reacting with the α-β unsaturated carboxylic acid polymer,
wherein the second paste includes at least a fluoroaluminosilicate glass powder and a polymerizable monomer not having an acid group,
wherein one or both of the first and second pastes include a photopolymerization catalyst,
wherein the mixing tip comprising:
a housing internally having a mixing element;
a paste guiding member being fixed to an end part positioned at the opposite side to an ejection port of the housing, and including a pair of paste inlets attached respectively to nozzles of the two syringes and flow path parts for guiding the pastes extruded from the respective syringes into the housing from the paste inlets; and
a locking member being fixed on or integrally formed on the paste guiding member or the housing, and having a pawl part to be locked with a locking hole formed on the syringe holder and a projection part to be inserted to a hole part bored on the syringe holder or having a pair of pawl parts to be inserted to and locked with two locking holes formed on the syringe holder, and
wherein the housing and the paste guiding member are made of a colored plastic material, which does not transmit a light having a wavelength photo-activating the photopolymerization catalyst but transmit light except the light having the photo-activating wavelength.

2. The mixing tip as claimed in claim 1,
wherein each pawl part includes a releasing mechanism for releasing a locking state of the syringe holder and the pawl part.
